# EUROPEAN PATENT APPLICATION

(11) **EP 1 110 445 A1**
(43) Date of publication of application: **27.06.2001**
(21) Application number: 00311357.8
(22) Date of filing: 19.12.2000
(51) Int. Cl.: A01F 12/00, G01N 27/04

(54) **Method of assessing maturity of vining peas during harvesting**

(30) Priority: 21.12.1999 EP 99310361
(71) Applicant: UNILEVER PLC, London EC4P 4BQ (GB); UNILEVER N.V., 3013 AL Rotterdam (NL)
(72) Inventor: Parmenter, Peter Frank, Bradwell, Great Yarmouth, NR31 9DJ (GB)
(74) Representative: Evans, Jacqueline Gail Victoria

(57) **Abstract**

A method of assessing maturity of vining peas during harvesting wherein said method comprises the steps ;
(i) harvesting pea material with a pea harvester;
(ii) assessing maturity of vining peas in said harvested pea material with a finometer cell;
(iii) compiling data from step (ii) with a data recording and display means
wherein said steps are repeatably performed at the harvest site.

## Description

### Field of the Invention

The present invention relates to a method and apparatus for measuring maturity of a crop. More particularly the invention relates to a method and apparatus for measuring crop maturity before the crop reaches the processing plant which may be used in the harvesting and processing of vegetables or soft fruits.

The invention may be seen to be particularly applicable to the harvesting and processing of peas, especially those marketed as fresh frozen peas.

### Background to the invention

When considering the harvesting and processing of peas that are to be marketed as fresh frozen peas it is important that the degradation in the quality of the peas, that inevitably occurs between harvesting and freezing, is kept to a minimum. This therefore necessitates that the time period between harvesting and freezing is kept as short as possible. For example, we ensure that such time is less than 2½ hours for fresh frozen peas.

The time period during which a pea crop is at optimal maturity and therefore most suitable for harvesting is known as the 'harvest window'. This is exceptionally short in the case of vining peas that are to be marketed as fresh frozen peas, typically in the range of from a day, down to a few hours depending on weather conditions. Harvesting at optimal maturity is a key element in achieving the highest quality peas , therefore being able to accurately assess maturity is critical to commercial pea production.

The assessment of maturity of a pea crop will provide data that plays a key role in assigning a quality grade to a load of harvested peas. The quality assigned to a pea load will determine the type of product that the peas will be destined for. Peas with the most favourable characteristics are assigned an F grade and will usually go to premium frozen products.

Assessing pea maturity by the measurement of alcohol insoluble solids has been used since the early 1930's. This chemical technique is unsuited to modern harvesting time regimes due to the 3 to 4 hours that it takes to complete. Moreover there is a greater tendency for variation in accuracy than with instrumental methods.

The physical properties of peas have also been used to assess the maturity thereof; in the " brine floatation test" salt solutions of various specific gravity's are utilised to separate peas according to maturity. While its simplicity is attractive this method has also been found to suffer considerable variation in accuracy.

An alternative means for assessing pea maturity in the prior art has been through the use of a finometer. This comprises a free standing piece of equipment containing a series of probes that are descended into a pea filled vessel by way of manual winding mechanism. The force of resistance to the crushing action of the probes is measured and the container must then be manually emptied and refilled with a subsequent sample. The conventional finometer design is considered to be an inaccurate means of assessing maturity and is not used for commercial pea maturity measurements.

Commercial assessment of pea maturity has conventionally been made by a combination of tasting the crop and measurement of a cleaned pea sample with a device called a tenderometer, at the processing plant. A tenderometer mechanically assesses the maturity of the peas by way of the resistance that is exerted by the peas to the cutting action of the device.

The conventional use of tenderometers in pea processing for an individual sample gives the most accurate assessment of maturity, and is consequently the most favoured approach in the art. For reasons of practicality, however, only a low number of pea maturity readings can be made, typically no more than two or three readings for around 4 tonnes of peas. Furthermore the samples that are taken are usually restricted to the peas that arrive at the plant on the surface of the load. With such a low number of readings poorly distributed amongst the load, the accuracy with which they reflect the maturity of the pea load as a whole can be highly insufficient.

Methods of using such factory based tenderometers for assessing pea maturity also give rise to a time delay before feedback can reach the field on the maturity of the peas being harvested. Measurement is made on arrival of a pea load at the factory and information is then fed back to the harvest site. The data provided by the tenderometer analyses forms the basis for a decision as to whether harvesting should continue. It would be clearly desirable for this feedback to be generated more effectively so that peas can be harvested more time efficiently or alternatively left for further ripening.

Tenderometers suffer from the further practical disadvantage that the pea sample must be clean i.e. not contain stones, or other harvest debris otherwise inaccurate results are achieved.

There is therefore a need to develop a system that can monitor pea maturity more effectively and time efficiently, providing a greater degree of accuracy over the whole pea load. It would be desirable to have a system for monitoring peas during harvesting to allow rapid feedback on the maturity of the crop. This would mean a pea load can be assigned a quality grade on the basis of maturity measurements, before arriving at the processing plant, allowing priority at the plant to be given to those loads of optimal maturity, highest quality and therefore greatest commercial value.

The technical problem to be solved by the present invention therefore relates to the accurate assessment of pea maturity before the arrival of a load at a processing plant. More particularly the problem requires a system which can accurately assess pea maturity throughout a pea load.

It has been found that a solution to this problem resides in a system for continuous and accurate monitoring of the maturity of peas as they are harvested in the field. Furthermore the solution resides in a use of equipment which has never been used for the present purpose of continuous in-field assessment of maturity and has therefore required considerable technical modification.

### Terms and definitions

For the purpose of the present invention, vining peas are considered to be immature pea seeds and typically giving maturity measurements in the range of 70 to 140 tenderometer units (TR), preferably 90 to 125 TR.

It is recognised that the pea harvester for use in the present invention may also be termed a pea viner and would be commercially available from a number of suitable suppliers such as FMC or Ploeger.

The term harvested material, reflects that the harvested matter will not consist solely of vining pea seeds. Pea shell fragments, stalk and foreign bodies such as stones, will invariably be collected with the pea seeds. At least 85% and more preferably 90% of the material harvested will however be pea seeds.

### Brief description of the invention

The present invention provides a novel finometer cell which may be used in the pea field during harvesting as part of an integrated system involving a harvester and data recording and display means. This system allows a high sample frequency in a pea load to be achieved during harvesting which leads to a accurate assessment of maturity for that load and providing real-time information that can be immediately fed to the processing plant.

It is therefore within a first aspect of the invention to provide a method of assessing maturity of vining peas during harvesting said method comprising the steps;
(i) harvesting pea material with a pea harvester;
(ii) assessing maturity of vining peas in said harvested pea material with a finometer cell;
(iii) compiling data from step (ii) with the data recording and display means;
wherein said steps are repeatably performed at the harvest site.

A first embodiment of the invention comprises a method as described above wherein the assessment of maturity with the finometer cell comprises the steps;
(i) filling one or more sample pots with a sample of harvested material;
(ii) inserting one or more probes into said one or more sample pots, wherein the probes are operably connected to a strain gauge capable of measuring the resistance of a particular sample;
(iii) compiling the resistance measurement data of step (ii) with the data recording and display means;
(iv) emptying the harvested material from the sample pot after step (ii), in preparation for the next filling step (i)

Preferably the harvest material is emptied from the sample pot in step (iv) by a clearing means comprising a removable base to the sampling pot and a plug, wherein said plug conforms in shape to the internal dimensions of said sampling pot and thus can be inserted therein when said base is removed, to empty the contents thereof.

In order to effectively and accurately assess the maturity of the pea seeds of the pea material and to allow mapping of the in-field variation in maturity, in a preferred embodiment, at least 10 resistance measurements are taken per tonne of harvested pea material. In order to avoid excessive waste of material in the sampling procedure, it is also preferred that the number of measurements is less than 30 per tonne. Most preferably the frequency of resistance measurements is in the range of 15 to 25 measurements per tonne.

In a most preferred embodiment performance of the above described method is carried out in an substantially automated manner.

A second aspect of the present invention relates to a finometer cell suitable for the automated assessment of the maturity of vining peas in harvested pea material during harvesting. A finometer cell according to the invention comprises at least one sample pot, one or more probes operably linked to a strain gauge and a clearing means. The interaction of the probes with the strain gauge of the finometer cell measures the resistance of a particular sample within the sample pot and provides a resistance measurement from which maturity can be assessed.

In a preferred embodiment the finometer cell of the invention comprises a single sample pot which is capable of adopting a plurality of positions to allow the steps of; filling with harvested material, measurement of resistance with said probes and strain gauge and emptying with one of said clearing means.

In a further preferred embodiment the clearing means of the finometer cell for emptying the harvest material from the sample pot comprises a removable base to the sample pot and a plug, wherein said plug is insertable into the sample pot.

In a third aspect, the invention relates to the use of a finometer cell as described above in the automated assessment of maturity of vining peas in harvested pea material during harvesting.

In a fourth aspect, the invention relates to an apparatus for assessing the maturity of vining peas during harvesting, wherein said apparatus comprises a pea harvester, a finometer cell and a data recording and display means, wherein said components form a single and preferably automated device.

A preferred embodiment comprises an apparatus for assessing the maturity of vining during harvesting wherein said finometer cell comprises at least one sampling pot, one or more probes operably connected to a strain gauge and at least one clearing means.

In a further preferred embodiment the apparatus according to the invention has a clearing means for emptying the sample pot which comprising a removable base to the sampling pot and a plug, wherein said plug, preferably conforming in shape to the internal dimensions of said sampling pot, is insertable therein.

A further aspect of the invention relates to the use of an apparatus as described above in a methods of assessing the maturity of vining peas during harvesting disclosed hereabove.

### Detailed description

The assessment of pea maturity is conducted using a finometer cell and a data recording and display means. The finometer cell comprises one or more sample pots and a series of probes operably connected to a strain gauge. Preferably the sampling pot(s) is capable of adopting a plurality of positions to allow the steps of filling with harvest material, measurement of resistance and emptying by way of a clearing means to occur with maximal efficiency.

The sample pots when filled with harvested pea material are positioned beneath the series of probes. These probes are then declined by an actuator into the said sample pot(s) wherein a strain gauge by measuring the resistance to the downward motion of the probes provides data from which maturity of the vining peas in the harvested material can be assessed. The data recording means used in the invention compiles the resistance voltage recordings from the strain gauge and uses it to generate a resistance profile for any particular sample. Preferably the signal of the strain gauge is converted to a single value accorded to the peak resistance i.e. peak voltage is taken as the indicator of resistance and thus maturity for a particular sample, however it is recognised that the totality of the resistance profile for a sample could be used as indication of maturity.

It has been found that where a large number of pods or stalks enter the sample pot during the filling stage this can cause inaccurate readings. This problem has been minimised by incorporating a sieve device into the finometer cell which prevents much of the pea debris from entering the sample pot. Preferably this sieve takes the form of a rotating disk that is positioned upstream from the sample pot in the flow of harvested material passing into the hopper. The device preferably consists of a stainless steel disk with holes to provide a centrifugal sieving effect when rotating. When in use the disk allows peas to pass though the holes of the disk into a sample pot below, while the larger stalks and pods pass over. Preferably the disk does not remain in the flow of harvested material continuously and is therefore pivotally mounted to enable it to swing out into the flow when a sample is to be taken.

Resistance recordings can be averaged over a particular time period or over a particular weight of harvested material. Whereas a single measurement cannot reliably provide an accurate indication of maturity, it has been shown that by sampling at a frequency of greater than 10 measurements per tonne and calculating mean readings, the finometer cell of the present invention can provide data from which an accurate assessment of pea maturity, that is comparable with the accuracy of a tenderometer, can be derived. A rolling mean reading for the maturity of samples over a period of time is preferably continuously displayed to the operator. The frequency of the sampling is preferably computer controlled and may be altered by the operator to suit a particular harvest regime.

In order to further improve the accuracy of measurement the data recording means may be set to filter out those results which appear distant from the calculated mean, to therefore eliminate freak results. Typically maturity readings having a value of less than 35Kg will be indicative of an error in sampling wherein the pot is only partially filled with harvested material. Similarly where a maturity reading is in excess of 90Kg this tends to represent a sample that has a large number of pods and therefore should be excluded from the results.

As the resistance of a sample is detected by the strain gauge and converted to a numerical value by the data-recording means it can be transferred to a visual display means for viewing by the machine operator. This allows a real-time information on the maturity of the crop to be available to the driver which will indicate whether a field is ready for harvesting and if so the quality grade, based on the maturity assessment from resistance readings, that be assigned to the crop. Furthermore this information can be forwarded to the processing plant so that prioritisation as to the processing order of the loads can be made based on the maturity and thus predicted quality of the arriving loads.

During measurement of resistance it is preferable not to descend the series of probes to the point of contact with the base of the sample pot as this will risk damaging the probes on any debris within the harvest material such as stones which have been pushed towards the bottom of the pot. It has been found, however, that the further from the base the probes descend the less accurate and repeatable the resistance measurements become. The applicants believe it would be within the ability of the person skilled in the art to establish the predetermined distance to which the probes are inserted to achieve maximal accuracy and minimal risk of damage thereto.

Differentials in maturity of the crop across an individual field will also become apparent from the continual real-time monitoring of maturity. In the case of peas, the short harvest window for achieving optimal maturity makes variation across a field a more significant influence on quality in the harvested load. This maturity data may therefore be used as a basis for separating the crop as it is harvested into batches of similar maturity and therefore similar quality. Compiling data on maturity differentials across a crop field can be used to develop field specific pea maturity maps which can be used to dictate harvest regimes that ensure optimal maturity is achieved in the harvested product.

Where maturity differentials are identified across the field the techniques of precision farming known in the art may be used to bring about a more uniform maturity at harvest, which will in turn increase the yield of optimal quality vining peas.

Once a resistance reading has been conducted the finometer cell must be emptied and refilled to allow subsequent measurements to be taken at the desired frequency. Emptying may be carried out using a clearing means to expel the contents of the sample pot, preferably the sample pot of the finometer cell has a removable base that allows the contents to be expelled using a plug, once the resistance has been measured.

The clearing means typically comprises a plug that conforms to the internal dimensions of the pot and may also have proximity sensors integral with its structure to prevent collision with the actual sides of the pot. Emptying and refilling is preferably conducted in an entirely automated manner as controlled by a series of coordinating actuators which are driven by a computer controlled precision hydraulics system.

### Example

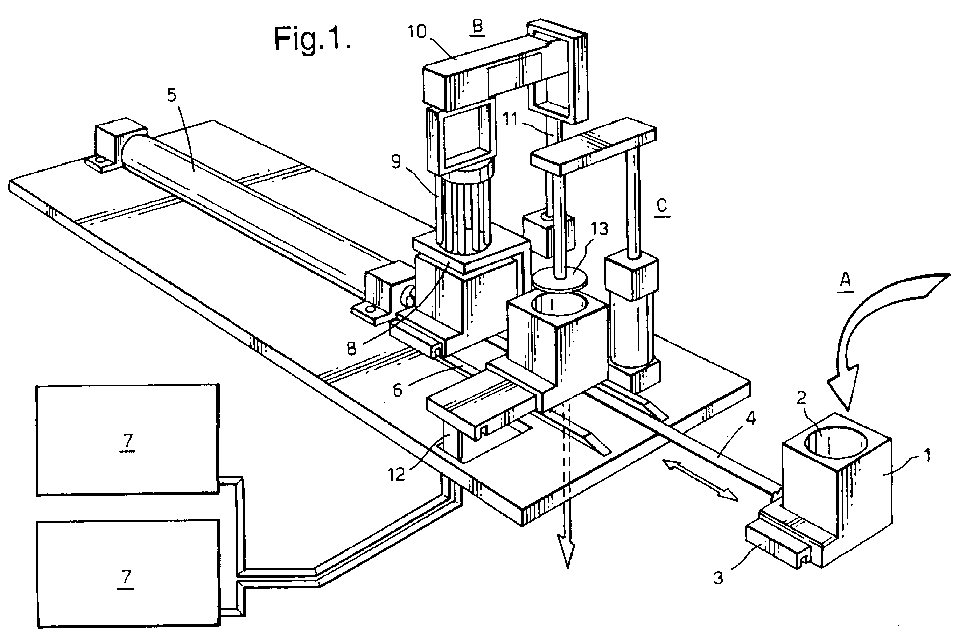

By way of example a finometer cell of the present invention will be more particularly described with reference to the accompanying drawing, Figure 1.

The finometer cell preferably comprises a sampling pot which can adopt a plurality of different positions that allow sampling (A), testing (B) and emptying (C).

The sampling pot comprises a vessel (1) with a opening at the top (2) and a slidably removable base (3). Said sampling pot is connected by way of a connecting limb (4) to a main positioning actuator (5) which directs horizontal movement of the sampling pot along support rails (6).

The drawing in Figure 1 depicts the sampling pot in three different positions demonstrating the three different stages involved in each measurement cycle.

In position (A) the sampling pot is filled with harvested material, once full the main positioning actuator as directed by a computer controlled precision hydraulic system (7) and by way of the connecting limb (4) moves the sampling pot into the testing position (B). This horizontal movement slides the sampling pot under the stripper plate (8) and in doing so removes excess harvested material from above the opening rim of the sampling pot.

Once in the testing position (B) a plurality of probes (9) attached to the strain gauge (10) are descended by an actuator (11) into the filled sample pot. In so doing the resistance to a downward force applied by the actuator (11) is measured by the strain gauge (10) and recorded by a data recording means (not shown). The probes descended to a predetermined distance from the base of the sample pot.

The probes (9) are then similarly withdrawn from the sample pot by the actuator (11) and the sample pot is directed by the actuator (5) to the emptying point at position (C). At the emptying point the removable base interacts with a lever (12) which under the influence of a further actuator slides the base horizontally, providing an opening in the base of the sampling pot (1).

At this stage a clearing means preferably in the form of a clearance plug (13) is descended into the vessel of the sampling pot to eject the tested sample of harvested pea material. The clearing means is then withdrawn, the base of the sampling pot repositioned and the sample pot is transferred to position A for receipt of a further sample of harvested pea material.

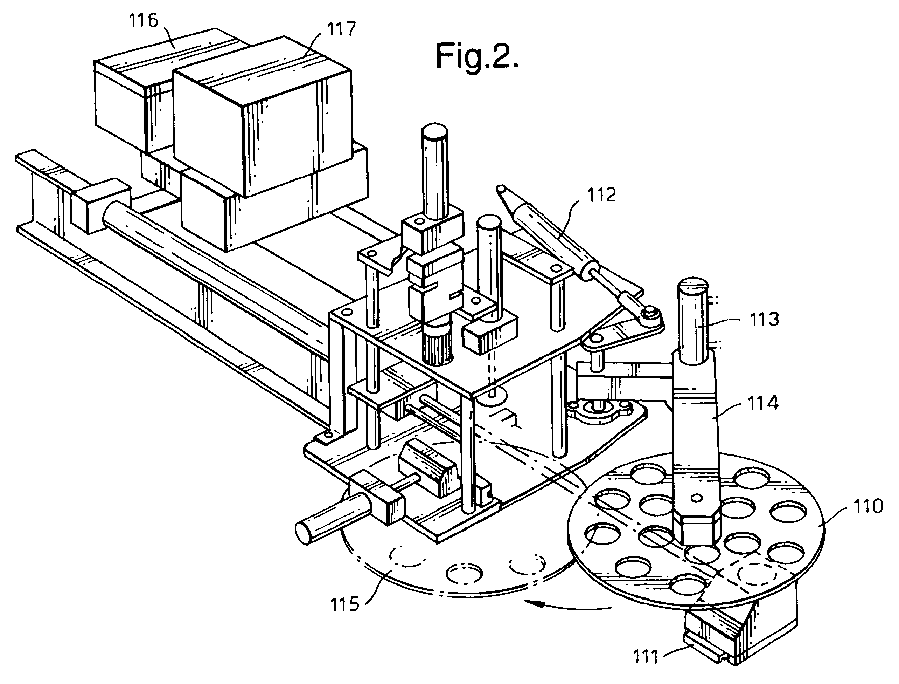

Figure 2 illustrates a further embodiment of the finometer cell of the invention. This example shows the additional feature relating to the disk sieve (110) located in the operating position above the sample pot (111). This sieve is pivotally mounted and movable into position by the positioning hydraulic cylinder (112). The hydraulic motor (113) drives the drive arm (114) for the rotation of the sieve when in position above the sample pot. When not in use the disk sieve is moved by the position cylinder (112) to a position (115) which is out of the flow of harvested material into the pea hopper. In figure 2 the electric terminal and main hydraulic computer control system have been positioned at (116) and (117) respectively.

## Claims

1. A method of assessing maturity of vining peas during harvesting wherein said method comprises the steps;
(i) harvesting pea material with a pea harvester;
(ii) assessing maturity of vining peas in said harvested pea material with a finometer cell;
(iii) compiling data from step (ii) with a data recording and display means
wherein said steps are repeatably performed at the harvest site.

2. A method according to claim 1 wherein the assessment of maturity with the finometer cell comprises the steps ;
(i) filling one or more sample pots with a sample of harvested material;
(ii) inserting one or more probes into said one or more sample pots wherein the probes are operably connected to a strain gauge capable of measuring the resistance of a particular sample;
(iii) compiling the resistance measurements of step (ii) with the data recording and display means;
(iv) emptying the harvested material from the sample pot after step (ii) in preparation for the next filling step (i).

3. A method according to claim 2, wherein the harvested material is emptied from the sample pot in step (iv) by a clearing means comprising a removable base to the sample pot and a plug, wherein said plug is inserted into the sample pot when the base is removed.

4. A method according to any one of the preceding claims, wherein at least 10 maturity measurements are taken per tonne of harvested pea material.

5. A method according to any one of the preceding claims, wherein said method is automated.

6. A finometer cell for assessing maturity of vining peas in harvested pea material comprising at least one sample pot, one or more probes operably connected to a strain gauge and at least one clearing means.

7. A finometer cell according to claim 6, wherein said sample pot is capable of adopting a plurality of positions to allow filling, measurement of resistance and emptying.

8. A finometer cell according to either claim 6 or claim 7, wherein said clearing means comprises a removable base to the sample pot and a plug, wherein said plug is insertable into the sample pot.

9. Use of a finometer cell according to any one of claims 6 to 8, in the automated assessment of maturity of vining peas in harvested pea material during harvesting.

10. Apparatus for assessing the maturity of vining peas during harvesting comprising a pea harvester, a finometer cell and a data recording and display means, wherein said components form a single automated device.

11. Apparatus according to claim 10, wherein said finometer cell comprises at least one sample pot, one or more probes operably connected a strain gauge and at least one clearing means.

12. Apparatus according to claim 11 , wherein said clearing means comprises a removable base to the sample pot and a plug, wherein said plug is insertable into the sample pot.

13. Use of an apparatus according to any one of claims 10 to 12 in a method according to any one of claims 1 to 5.
